# EUROPEAN PATENT SPECIFICATION

(11) **EP 1 771 178 B1**
(45) Date of publication and mention of the grant of the patent: **05.08.2009**
(21) Application number: 05778429.0
(22) Date of filing: 29.07.2005
(51) Int. Cl.: A61K 31/553, A61P 3/04, A61P 1/04, A61P 9/00, A61P 13/00, A61P 17/00, A61P 19/00, A61P 25/00, A61P 31/00, A61P 33/00, A61P 35/00, A61P 37/00

(54) **USE OF K-252A AND KINASE INHIBITORS FOR THE PREVENTION OR TREATMENT OF HMGB1-ASSOCIATED PATHOLOGIES**
VERWENDUNG VON K-252A UND KINASEHEMMERN ZUR VORBEUGUNG UND BEHANDLUNG VON HMGB1-ASSOZIIERTEN PATHOLOGIEN
EMPLOI DE K-252A ET D'INHIBITEURS DE KINASE POUR LA PREVENTION OU LE TRAITEMENT DE PATHOLOGIES ASSOCIEES A HMGB1

(30) Priority: 29.07.2004 US 591880 P; 27.01.2005 US 647007 P
(43) Date of publication of application: 11.04.2007
(73) Proprietor: Creabilis Therapeutics S.P.A., 10010 Colleretto Giacosa (IT); Bio3Research Srl., 20121 Milano (IT)
(72) Inventor: FUMERO, Silvano, I-10015 Ivrea (TO) (IT); PILATO, Francesco, P., I-20121 Milano (IT); BARONE, Domenico, I-10129 Torino (IT); BERTARIONE, RAVA, Rossa, Luisa, I-10018 Pavone Canavese (TO) (IT); MAINERO, Valentina, I-10015 Ivrea (TO) (IT); TRAVERSA, Silvio, I-10010 Palazzo Canavese (TO) (IT)
(74) Representative: Weiss, Wolfgang
(86) International application number: PCT/EP2005/008258
(87) International publication number: WO 2006/010628

(56) References cited:
- EP-A- 0 641 566
- EP-A- 1 121 932
- EP-A- 1 364 628
- WO-A-00/30651
- WO-A-01/12609
- WO-A-96/11933
- WO-A-96/13506
- WO-A-97/46565
- WO-A-97/49406
- WO-A-98/39007
- WO-A-98/42339
- WO-A-99/62503
- WO-A-02/057473
- WO-A-02/074242
- WO-A-03/099221
- WO-A-20/04024093
- WO-A-20/04032709
- WO-A-20/04038422
- WO-A-20/04044001
- WO-A-20/04060318
- WO-A-20/04084891
- WO-A-20/04087941
- WO-A-20/04112794
- WO-A-20/05014003
- WO-A-20/05074921
- DE-A1- 19 917 990
- US-A- 4 555 402
- US-A- 5 264 431
- US-A- 5 633 235
- US-A- 5 658 898
- US-A- 5 726 164
- US-A- 5 977 184
- US-A- 5 998 386
- US-A1- 2001 025 059
- US-A1- 2004 018 996
- US-A1- 2004 044 405
- US-A1- 2004 054 180
- PATENT ABSTRACTS OF JAPAN vol. 014, no. 137 (C-0702), 15 March 1990 (1990-03-15) & JP 02 009819 A (ASAHI CHEM IND CO LTD; others: 01), 12 January 1990 (1990-01-12)
- PATENT ABSTRACTS OF JAPAN vol. 011, no. 394 (C-465), 23 December 1987 (1987-12-23) & JP 62 155284 A (KYOWA HAKKO KOGYO CO LTD), 10 July 1987 (1987-07-10)
- PATENT ABSTRACTS OF JAPAN vol. 013, no. 124 (C-580), 27 March 1989 (1989-03-27) & JP 63 295589 A (KYOWA HAKKO KOGYO CO LTD), 1 December 1988 (1988-12-01)
- PATENT ABSTRACTS OF JAPAN vol. 1995, no. 11, 26 December 1995 (1995-12-26) & JP 07 223958 A (ASAHI CHEM IND CO LTD; others: 01), 22 August 1995 (1995-08-22)
- KATSUMI INOUE ET AL: "HMGB1 expression by activated vascular smooth muscle cells in advanced human atherosclerosis plaques" CARDIOVASCULAR PATHOLOGY, vol. 16, 2007, pages 136-143,
- [Online] Retrieved from the Internet: URL:atvb.ahajournals.org> [retrieved on 2008-08-10]
- ANNALISA PORTO ET AL: "Smooth muscle cells in human atherosclerotic plaques secrete and proliferate in response to high mobility group box 1 protein" FASEB JOURNAL, vol. 20, no. 14, 2 December 2006 (2006-12-02), pages E1955-E1963,
- "The nuclear factor HMGB1 mediates hepatic injury after murine liver ischemia-reperfusion" JOURNAL OF EXPERIMENTAL MEDICINE, vol. 201, no. 7, 4 April 2005 (2005-04-04), pages 1135-1143,
- WATANABE TAIJI ET AL: "The role of HMGB-1 on the development of necrosis during hepatic ischemia and hepatic ischemia/reperfusion injury in mice" JOURNAL OF SURGICAL RESEARCH, vol. 124, no. 1, March 2005 (2005-03), pages 59-66,

## Description

The present invention relates to the use of K-252a, a physiologically active substance produced by microorganisms, or/and a kinase inhibitor and of its salts or synthetic and/or chemically modified derivatives for the prevention or treatment of HMGB1-associated pathologies. More particularly, the present invention relates to the use of K-252a or/and a kinase inhibitor for the prevention or treatment of restenosis.

Recent researches in the field of sepsis and inflammation have led to an improved understanding of the pathogenic mechanisms and events underlying their clinical onset and development. In the early stages of sepsis, for instance, bacterial endotoxins stimulate cells of the innate immune system which release pro-inflammatory cytokines (TNF, IL-1α and IL-6). These early cytokines, in turn, induce the release of a later-acting downstream mediator - identified as the known protein HMGB1 - that triggers the pathological sequelae mediated by the subsequent release of cytokines like TNF, IL-1α, IL-1β, IL-1Ra, IL-6, IL-8, etc., leading to a multisystem pathogenesis or to a lethal systemic inflammation.

The HMGB1 protein belongs to the family of high mobility group (HMG) proteins. HMG proteins, so called due to their high electrophoretic mobility in polyacrylamide gels, are the most ubiquitous non-histone proteins associated with isolated chromatin in eukaryotic cells. These proteins play a generalized "architectural" role in DNA bending, looping, folding and wrapping since they either distort, bend or modify DNA structures complex with transcription factors or histones. The high mobility group 1 (HMGB1) protein is usually a nuclear factor, in particular a transcriptional regulatory molecule causing DNA bending and facilitating the binding of several transcriptional complexes.

Extracellularly released HMGB1 acts as a potent cytokine and as an extremely potent macrophage-stimulating factor. HMGB1 acts directly by binding to the cell membrane inducing signaling and chemotaxis, having a chemokine-like functions, and further acting indirectly by up-regulating the expression and secretion of pro-inflammatory cytokines. This makes extracellular HMGB1 protein a potent chemotactic and immunoregulatory protein which promotes an effective inflammatory immune response.

Furthermore, other proteins belonging to the family of HMG-proteins and able to bend DNA are released together with HMGB1 in the extracellular medium. These proteins are inter alia HMGB2, HMGB3, HMG-1L10, HMG-4L and SP100-HMG. They share with HMGB1 highly homologous amino acid sequences. Like HMGB1, they trigger/sustain inflammatory pathologies interacting with the same receptors and leading to the same downstream pathways of interaction.

The release of HMGB1 by injured and necrotic cells has been demonstrated to actively mobilize rat smooth muscle cells (RSMC) *in vitro* (1) and to trigger inflammation in vivo (2).

In healthy cells, HMGB1 migrates to the cytoplasm both by passive and active transport. However, all cultured cells and resting monocytes contain the vast majority of HMGB1 in the nucleus, indicating that in baseline conditions import is much more effective than export. Cells might transport HMGB1 from the nucleus by acetylating lysine residues which are abundant in HMGB1, thereby neutralizing their basic charge and rendering them unable to function as nuclear localization signals. Nuclear HMGB1 hyperacetylation determines the relocation of this protein from the nucleus to the cytoplasm (in the fibroblasts, for example) or its accumulation into secretory endolysosomes (in activated monocytes and macrophages, for example) and subsequent redirection towards release through a non-classical vesicle-mediated secretory pathway. HMGB1 secretion by already activated monocytes is then triggered by bioactive lysophosphatidylcholine (LPC), which is generated later in the inflammation site from phosphatidylcholine through the action of the secretory phospholipase sPLA2, produced by monocytes several hours after activation. Therefore, secretion of HMGB1 seems to be induced by two signals (Bonaldi et al., 2003) and to take place through three steps: 1) at first, an inflammatory signal promotes HMGB1 acetylation and its relocation from the nucleus to the cytoplasm (step 1) and storage into cytoplasmic secretory vesicles (step 2); then, a secretion signal (extracellular ATP or lysophosphatidylcholine) promotes exocytosis (third step) (Andersson et al., 2002; Scaffidi et al. 2002; Bonaldi et al., 2003; Friedman et al.,2003; Gardella et al., 2002).

Released HMGB1 has been identified as one of the ligands binding to the RAGE receptor. This receptor is expressed in most cell types, and at a high level mainly in endothelial cells, in vascular smooth muscle cells, in monocytes and monophages and in mononuclear phagocytes. Recognition involves the C-terminal of HMGB1. The interaction of HMGB1 and RAGE triggers a sustained period of cellular activation mediated by RAGE up-regulation and receptor-dependent signaling. In particular, the interaction of HMGB1 and RAGE activates several intracellular signal transduction pathways, including mitogen-activated protein kinases (MAPKs), Cdc-42, p21 ras, Rac and the nuclear translocation factor κB (NF- κB), the transcription factor classically linked to inflammatory processes (Schmidt et al.,2001).

According to several experimental evidences, released HMGB1 may also interact with the receptors belonging to the family of the Toll-like receptors (TLR), e.g. with the subclasses TLR2, TLR4, TLR7, TLR8 or/and TLR9.

Furthermore, HMGB1 may also interact with the functional N-terminal lectin-like domain (D1) of thrombomodulin. Due to the ability of the functional D1 domain of thrombomodulin to intercept and bind circulating HMGB1, the interaction of the HMGB1 with the RAGE-receptors and the Toll-like receptors is prevented.

Structurally, the HMGB1 protein is a ca. 25 kDa protein with a highly conserved sequence among mammals, whereby 2 out of 214 amino acids have conservative substitutions in all mammalian species. HMGB1 is ubiquitously present in all vertebrate nuclei and, in particular, can be found in fibroblasts, neurons, hepatocytes, glia and in cells derived from hematopoietic stem cells, including monocytes/macrophages, neutrophils and platelets. The HMGB1 molecule has a tripartite structure composed of three distinct domains: two DNA binding domains called HMG Box A and Box B, and an acid carboxyl terminus, making it bipolarly charged. The two basic DNA-binding domains, called box-A and box-B, are able to recognize and bind DNA with high affinity and interact with several transcription factors and nuclear steroid receptors. They play a crucial role not only in transcription processes, but also in apoptosis (programmed cell death) induction (3-5).

Recently, it was shown that, unlike injured or necrotic cells which release HMGB1 by simple diffusion and thereby trigger inflammation, apoptotic cells avidly retain HMGB1 bound to chromatin remnants even after their eventual lysis. It has also been argued that extracellular HMGB1 is primarily a signal of tissue damage and monocytes and macrophages have "learned" to mimic an ancient alarm signal. Besides, very recently HMGB1 was shown to induce migration and proliferation of both adult and embryonic mesoanglioblasts and smooth muscle cells (2 and WO 02/074337).

K-252a (molecular weight 467,5) is a glycosylated indole carbazole isolated for the first time in 1986 (6) from *Nocardiopsis sp*. (US 4,555,402 and WO 97/38120 - EP 0 834 574 B1). Since it is a lipophilic molecule it is capable of crossing the membranes of living cells. K-252a is a non-specific inhibitor of the broad family of serine/threonine protein kinases such as pkA, pkC, pkG, myosin light chain kinase (7), CaM kinase II and is characterized by a nM affinity for NGF receptors (TrkA). The chemical structure of K-252a is depicted in Formula (I):

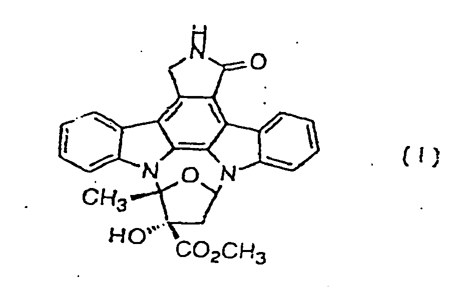

K-252a has anti-histamine releasing, anti-allergic effects (US 4,555,402) and an anti-proliferative effect on human prostatic carcinoma cell lines (8) and on human psoriatic keratinocytes (WO 2005/014003). The latter activity is due to TrkA-phosphorylation blockade and consequent NGF activity inhibition. Further, it has been shown that both human and rat smooth muscle cells express NGF and its receptor TrkA (9).

In JP 02 009819 it has further been shown that K-252a has a suppressing or relaxing activity on the contraction of blood vessels and is therefore expected to improve various symptoms which are associated with cerebral blood flow insufficiency, cerebral infarction, cerebral vasospam, stenocardia and coronary spasm.

Derivatives of K-252a are also disclosed as being useful in the treatment of a variety of diseases. In JP 62 155284 an O-methyl ester derivative of K-252 is shown as potentially useful in the prevention and treatment of allergies, tumors or diseases of the cardiovascular system. JP 63 295589 discloses a K-252a derivative as antiallergic agent, antithrombotic agent, antiinflammatory agent or antitumor agent. In WO 97/49406 it is shown that a K-252a derivatives might also be useful in treating peripheral neuropathies, central neuronal degeneration and cytokine overproduction, particulary Alzheimer's disease, Parkinson's disease and autoimmune and allergic conditions. EP 1 121 932 relates to therapeutic preparations of K-252a derivatives for eye diseases. In WO 97/46565 to the use of a K-252a derivative with PKC inhibitory activity for treating neurological disorders is disclosed. In WO 96/11933 a K-252a derivative for effecting the function and/or survival of a trophic factor responsive cell, particularly for treating inflammation, allergies, cancer, sepsis, MS, rheumatoid arthritis or psoriasis is disclosed. WO 96/13506 relates to the use of a K-252a derivative for the treatment of neurological disorders.

Surgical procedures during angioplasty frequently induce intima injury causing the damage and necrosis of a variety of cell types, including endothelial cells. This may result in restenosis, a condition characterised by reclosure of arteries - caused by re-proliferation and re-migration of blood vessel cells. Today, restenosis occurs in more than 20% of patients after surgical angioplasty and this condition requires a second surgery. Thus, there is a need for novel medicaments which are suitable for the prevention or treatment of restenosis.

In the present invention it has been demonstrated that K-252a (i) has a potent biological effect on HMGB1-induced smooth muscle cells migration and proliferation in response to the mechanical injury induced by surgical stent application and (ii) acts as an antagonist/inhibitor of the broad spectrum of pathological activities triggered and sustained/amplified by HMGB1 itself in its role of pro-inflammatory chemotactic chemokine and/or by the cascade of inflammatory cytokines induced by its release.

Further, it was surprisingly found that HMGB1 is secreted by smooth muscle cells in human atherosclerotic plaques (M. Bianchi, unpublished results).

Thus, a first aspect of the present invention relates to the use of K-252a or/and a salt or a derivative thereof for the preparation of a medicament for the prevention or treatment of an HMGB1-associated pathology, which is triggered and/or sustained by HMGB1 and/or HMGB1 homologous proteins selected from restenosis, atherosclerosis or ischemia-reperfusion injury, wherein said derivative is a synthetic and/or chemically modified compound, which is a compound having substituents on the ring system selected from C₁-C₄ alkyl groups, a compound wherein the methyl ester group has been replaced by another ester group, an amide group or by H or a cation, and/or a compound wherein the N-atom in the cyclic amide group is substituted with a C₁-C₄ alkyl group, and wherein the HMGB1 homologous proteins are HMGB2, HMGB3, HMG-1L10, HMG-4L or/and SP100-HMG. In this aspect, K-252a or/and a salt or a derivative thereof as defined above is administered in a therapeutically effective dose to a subject in need thereof in order to prevent or treat said HMGB1-associated pathologies.

In the context of the present invention, said HMGB1-associated pathologies are preferably pathologies associated with the non-acetylated or/and acetylated form of HMGB1 or/and associated with the non-acetylated or/and acetylated form of HMGB1 homologous proteins. Therefore, in the use of the present invention, said HMGB1-associated pathologies are preferably pathologies associated with the non-acetylated or/and with the acetylated form of HMGB1 or of HMGB1 homologous proteins as defined. In the method of the present invention for the prevention or treatment of the HMGB1-associated pathologies, HMGB1-associated pathologies are preferably pathologies associated with the non-acetylated or/and acetylated form of HMGB1 or of HMGB1 homologous proteins as defined herein.

In the context of the present invention, "HMGB1" includes the non-acetylated form or/and the acetylated form of HMGB1. Likewise "HMGB1 homologous proteins" include the non-acetylated form or/and the acetylated form of HMGB1 homologous proteins selected from HMGB2, HMGB3, HMG-1L10, HMG-4L or/and SP100-HMG.

The K-252a used in the context of the present invention can be obtained either by (i) extraction and purification from microorganism cells containing K-252a and which are obtained by culturing microorganisms capable of producing K-252a or/and by (ii) chemical synthesis (Wood et al., J. Am. Chem. Soc. 117:10413-10414, 1995). Microorganisms which produce K-252a and from which K-252a can be isolated belong preferably to the genus Nocardiopsis sp. and Saccaromyces sp.

Without wishing to be bound by theory, the circular dichroism data of the present invention and fluorescence data indicate that the inhibitory action of K-252a upon the HMGB1 activity does not necessarily depend on a direct interaction between K-252a and HMGB1. Although the mechanism by which K-252a inhibits HMGB1 activities has not yet been fully elucidated, it is more than likely that K-252a inhibits the activity of HMGB1 by inhibition of at least one kinase, such as a tyrosine kinase, a phosphokinase or/and a further kinase. Extracellular HMGB1 interacts with its membrane receptors, in particular RAGE and TLR receptors, triggering the initiation of a kinase cascade inside the cell. This cascade transports the information of the extracellular HMGB1 binding throughout the cytoplasm and in the nucleus, causing the cell to respond to the external stimulus. It is supposed that K-252a can block the cascade triggered by HMGB1 binding at different stages, and thus resulting in an overall inhibition of HMGB1 action on the cell.

The inventors of the present invention have tested the inhibition activity of K-252a on 67 human kinases and found that K-252a is capable of inhibiting several of these molecules. Indeed, 17 out of the 67 kinases tested showed an inhibition greater than 90% (cf. Example 5). Thus, K-252a has been identified as an agent for treating disorders associated with one or several kinases as shown in Table 1 and 2, for which an inhibition of at least 80 percent, preferably of at least 90 percent and more preferably of at least 95 percent has been found.

A disorder associated with a kinase is preferably a disorder associated with increased kinase activity in a diseased cell or organism compared to a non-diseased cell or organism. Kinase activity may be determined on transcript level (e.g. by measuring mRNA) or on protein level (e.g. by measuring amount and/or activity of protein).

It is further disclosed herein, as indicated above, that K-252a may be used with at least one further kinase inhibitor, an inhibitor selected from tyrosine kinase inhibitors or/and phosphokinase inhibitors. A tyrosine kinase inhibitor is an inhibitor of TrkA, TrkB, TrkC or/and an inhibitor of the subfamily of tyrosine kinase receptors including the Ron receptor, c-Met (receptor of HGF/scatter factor) and Sea receptors. A phosphokinase inhibitor is an inhibitor of PKA, PKC, or/and PKG. Another kinase inhibitor is an inhibitor of other kinases such as Raf kinase, Ras kinase, CaM kinase, MLC kinase, MAP kinase, MEK, ERK, JUN kinase or/and PI3Kγ.

There are kinase inhibitors known in the state of the art. Suitable known inhibitors of CaM kinase are calmodulin binding domain, Ca²⁺/calmodulin kinase II inhibitor 281-309, hypericin. Suitable known inhibitors of TrkA, TrkB or/and TrkC are CP 701, genistein, herbimycin, lavendustin, quercetin, radicicol. Suitable known inhibitors of MAP kinase are hymenialdisine, CP-1347, olomoucine, CC-401. Suitable known MLC kinase inhibitors are piceatannol, staurosporine, myosin light chain inhibitor peptide 18. Suitable known phosphatidylinositol-3 kinase (PI3Kγ) inhibitors are quercetin, wortmannin. Suitable known PKA inhibitors are staurosporine, KT-5720. Suitable known PKC inhibitors are staurosporine, bisindolylmaleimide I, calphostin C, and chelerytrine. Suitable known PKG inhibitors are staurosporine, H-7, H-9, and KT-5823.

In the present invention, K-252a may be employed in the form of a salt or/and derivative as defined.

Preferred K-252a salts or/and salts of kinase inhibitors are salts with pharmaceutically acceptable cations, e.g. alkaline or alkaline-earth cations or anions, e.g. inorganic anions or organic anions.

An HMGB1-associated pathology is a condition in a patient wherein an increased concentration of the HMGB1 protein and/or of HMGB1 homologous proteins in the acetylated or non-acetylated form is present in the biological fluids and tissues, compared to the concentration in normal subjects where these HMGB1 proteins are practically undetectable. The HMGB1-associated pathologies and/or the pathologies associated with HMGB1 homologous proteins are pathologies with a strong inflammatory basis or pathologies which result from the stimulation of cytokine such as TNF-alpha, IL-1, IL-6 etc., or pathologies which result from toxic events, such as intoxication, infection, burn, etc. In particular high concentrations of the HMGB1 protein and homologous proteins have been found and determined in plasma of patients with sepsis, in plasma and synovial fluid of rheumatoid arthritis patients, in brains of Alzheimer's disease patients, in plasma and tissues of melanoma patients, in plasma of systemic lupus erythematosus patients, in atherosclerotic plaques of atherosclerotic patients, etc. The determination and evidence of HMGB1 protein and/or homologous proteins in biological fluids and tissues, may be detected by common diagnostic tools known by the skilled person in the art, including for example detection by ELISA assays etc.

The HMGB1-associated pathologies according to the present invention are preferably pathological conditions mediated by activation of the inflammatory cytokine cascade induced by the HMGB1-chemokine and by the HMGB1-induced cascade of inflammatory cytokines selected from restenosis, atherosclerosis or ischemia-reperfusion injury, caused by, associated with and/or accompanied by HMGB1 protein release.

In an especially preferred embodiment, K-252a is used for the prevention or treatment of artherosclerosis and/or restenosis occurring during or after angioplasty. More preferably, the medicament is used for blocking, retarding and/or impairing connective tissue regeneration in restenosis during or after angioplasty.

By inhibiting HMGB1 activity, the migration and proliferation of smooth muscle cells (SMC) that occur during restenosis can be prevented and/or inhibited. SMCs are located in the tunica media where they are embedded in an extracellular matrix. In intact vessels, SMC cells are in contractile state and show a phenotype characterised by absence of cell division and migration which is responsible for vessel wall rigidity, elasticity maintenance and peripheral blood pressure control.

When the vessel endothelium is damaged, either after mechanical (scraped by stent insertion) or local inflammatory injuries, SMCs switch to a synthetic phenotype and undergo cell division and migration. The migration of SMCs from the tunica media to the tunica intima, resulting in intimal thickening, plays an important role in the pathophysiology of many vascular disorders, such as atherosclerosis and restenosis after coronary angioplasty. In the synthetic state, SMCs also produce higher amounts of extracellular proteinases, growth factors, cytokines and secrete a fibrous extracellular matrix. After vessel wall traumatic injury, the release of several growth factors and/or chemoattractants and cell proliferation inducers (HMGB1 and HMGB1 homologous proteins are one of the most relevant), either by activated circulating monocytes, macrophages and platelets, or by damaged endothelial cells, can induce the switch of SMC cells from the contractile to the synthetic phenotype and direct their migration towards the vessel intima. In the context of the present invention it was surprisingly found that HMGB1 and HMGB1 homologous proteins are secreted by smooth muscle cells in human atherosclerotic plaques. Thus, K252a or/and derivatives thereof as defined are suitable therapeutic agents in the prevention and/or treatment of restenosis, e.g. through systemic administration or/and through drug-eluting stents.

K-252a or/and derivatives thereof as defined may be used either alone or in combination with one or several further agents. In particular, K-252a or/and derivatives thereof as defined may be used in combination with at least one further agent capable of inhibiting an early mediator of the inflammatory cytokine cascade. For example, K-252a derivatives as defined thereof may be administered together with an agent capable of inhibiting early mediators of the inflammatory cytokine cascade, e.g. an antagonist or inhibitor of a cytokine selected from the group consisting of TNF, IL-1α, IL-1β, IL-Rₐ, IL-8, MIP-1α, MIF1β, MIP-2. MIF and IL-6.

The further agent used in combination with K-252a or/and derivatives thereof as defined may also be an inhibitor of RAGE, e.g. an antibody directed to RAGE, a nucleic acid or nucleic acid analogue capable of inhibiting RAGE expression, e.g. an antisense molecule, a ribozyme or a RNA interference molecule, or a small synthetic molecule antagonist of the interaction of HMGB1 with RAGE, preferably of the interaction of the non-acetylated or/and acetylated form of HMGB1 with RAGE, or soluble RAGE (sRAGE). The antibody to RAGE is preferably a monoclonal antibody, more preferably a chimeric or humanised antibody or a recombinant antibody, such as a single chain antibody or an antigen-binding fragment of such an antibody. The soluble RAGE analog may be optionally present as a fusion protein, e.g. with the Fc domain of a human antibody. The small synthetic molecular antagonist of the HMGB1 interaction with RAGE preferably has a molecular weight of less than 1000 Dalton. The small synthetic molecular antagonist preferably inhibits the interaction of RAGE with the non-acetylated form or/and with the acetylated form of HMGB1 and with the non-acetylated form or/and with the acetylated form of HMGB1 homologous proteins selected from HMGB2, HMGB3, HMG-1L10, HMG-4L or/and SP100-HMG.

Furthermore, the further agent may be an HMGB1 antagonist/inhibitor, e.g. an antibody against HMGB1, particularly against the HMGB1 Box-B or a fragment of HMGB1 which has antagonistic activity, e.g. a Box-A fragment. Suitable HMGB1 antagonists and inhibitors are disclosed in US 6,468,533, WO 02/074337 and US 2003/144201. The HMGB1 antagonist/inhibitor is preferably an antagonist/inhibitor of the non-acetylated or/and acetylated form of HMGB1.

The further agent used in combination with K-252a or/and derivatives thereof as defined may also be an inhibitor of the interaction of a Toll-like receptor (TLR), e.g. of TLR2, TLR4, TLR7, TLR8 or/and TLR9, with HMGB1, which inhibitor is preferably a monoclonal or polyclonal antibody, a nucleic acid or nucleic acid analogue capable of inhibiting TLR expression, e.g. an antisense molecule, a ribozyme or a RNA interference molecule, or a synthetic molecule preferably having a size of less than 1000 Dalton. The inhibitor may be a known inhibitor of a Toll-like receptor, in particular of TLR2, TLR4, TLR7, TLR8 or/and TLR9. The inhibitor preferably inhibits the interaction of the Toll-like receptor with the non-acetylated form or/and the acetylated form of HMGB1 and with the non-acetylated form or/and with the acetylated form of HMGB1 homologous proteins selected from HMGB2, HMGB3, HMG-1L10, HMG-4L or/and SP100-HMG.

In still another embodiment, the further agent used in combination with K-252a or/and derivatives thereof as defined is the functional N-terminal lectin-like domain (D1) of thrombomodulin. The D1 domain of thrombomodulin is able to intercept the non-acetylated form and/or the acetylated form of released HMGB1 and of released HMGB1 homologous proteins selected from HMGB2, HMGB3, HMG-1L10, HMG-4L or/and SP100-HMG, preventing thus their interaction with RAGE and Toll-like receptors. The D1 domain of thrombomodulin may be native or mutated in order to make it resistant to proteases.

The further agent may also be a synthetic double-stranded nucleic acid or nucleic acid analogue molecule with a bent shape structure, particularly a double-stranded bent DNA, PNA or DNA/PNA chimera or hybrid or a double-stranded cruciform DNA, PNA or DNA/PNA chimera or hybrid structure, capable of binding to the HMGB1 protein. Preferred nucleic acids and nucleic analogue molecules are disclosed in a co-owned and co-pending international patent application no. PCT/EP2005/007198 filed on 4 July 2005 (claiming the priority of US provisional application No. 60/584,678 filed on 2 July 2004). The synthetic double-stranded nucleic acid or nucleic acid analogue molecule with a bent shape structure is preferably capable of binding to the non-acetylated or/and to the acetylated form of HMGB1 and the non-acetylated or/and the acetylated form of HMGB1 homologous proteins selected from HMGB2, HMGB3, HMG-1L10, HMG4L or/and SP100-HMG.

The K-252a or/and a derivative thereof as defined is/are usually administered as a pharmaceutical composition, which additionally comprises pharmaceutically acceptable carriers, diluents and/or adjuvants.

The administration may be carried out by known methods, e.g. by injection, in particular by intravenous, intramuscular, transmucosal, subcutaneous or intraperitoneal injection and/or by oral, topical, nasal, inhalation, aerosol and/or rectal application, etc. The administration may be local or systemic.

Hence, the present invention further discloses a pharmaceutical composition comprising an effective amount of K-252a or/and a salt or a derivative thereof as defined as an active ingredient for the treatment of said HMGB1-associated pathologies and pharmaceutically acceptable carriers, diluents and/or adjuvants for the treatment of pathologies associated with the non-acetylated or/and the acetylated form of HMGB1 and/or of HMGB1 homologous proteins as disclosed.

The exact formulation, route of administration and dosage can be chosen by the individual physician in view of the patient's conditions. Administration may be achieved in a single dose or repeated doses at intervals. Dosage amount and interval may be adjusted individually in order to provide the therapeutical effect which results in amelioration of symptoms or a prolongation of the survival in a patient. The actual amount of a composition administered will, of course, be dependent on the subject being treated, on the subject's weight, the severity of the affliction, the manner of administration and the judgement of the prescribing physician. A suitable daily dosage will be between 0,001 to 10 mg/kg, particularly 0,1 to 5 mg/kg.

Such a pharmaceutical composition may be used for diagnostic or for therapeutic applications. For diagnostic applications, the compound may be present in a labelled form, e.g. in a form containing an isotope, e.g. a radioactive isotope or an isotope which may be detected by nuclear magnetic resonance. A preferred therapeutic application is blocking, retarding or reducing connective tissue regeneration.

K-252a or/and a salt or a derivative thereof as defined may be administered as a free compound and/or reversibly immobilized on the surface of the medical device. For this purpose, a medical device may be reversibly loaded with the active ingredient and, optionally, further agents, in particular by binding, embedding and/or absorbing the medicament molecules onto the surface of the medical device or on a coating layer on the surface of the medical device. After contacting the medical device with body fluid or body tissue, the reversibly immobilised compounds are liberated. Consequently, the coated medical devices act as drug delivery devices eluting the medicament, whereby the drug delivery kinetics can be controlled, providing an immediate release or a controlled, delayed or sustained drug delivery, for example. For a controlled, delayed or sustained release, the active agent can be embedded into nano- or microcapsules or a matrix coating, in particular a polymer matrix coating can be applied on a medical device, such as a stent. Coating technologies of medical devices are well known to the person skilled in the art.

Therefore, a further aspect of the present invention relates to a medical device reversibly coated or embedded with K-252a or/and a salt or a derivative thereof as disclosed. Preferably, the medical device is selected from surgical instruments, implants, catheters or stents, e.g. stents for angioplasty. Most preferably, the medical device according to the invention is a drug-eluting stent (DES).

Further, the present invention is explained in more detail in the following Figures and Examples.

### Brief description of the drawings.

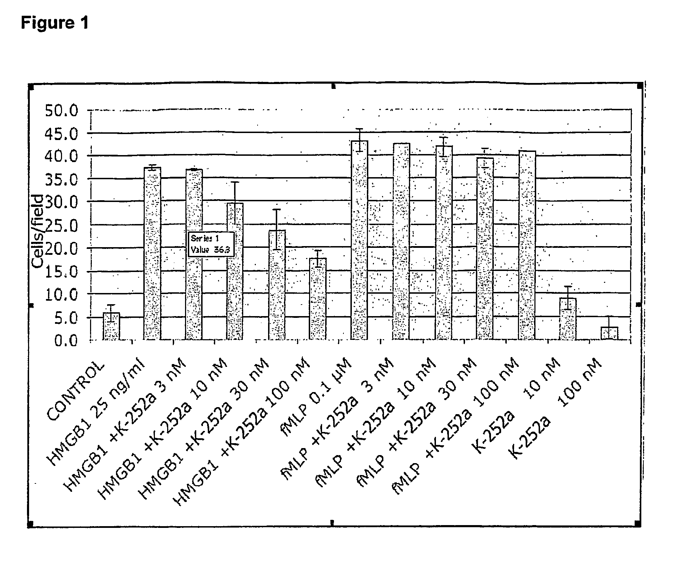

Figure 1 - Activity of K-252a in a chemotaxis assay.
   Inhibitory activity of K-252a on bovine aorta smooth muscle cells (BASMC) in a typical migration (chemotaxis) assay performed using modified Boyden chambers and two different chemoattractants: HMGB1 and fMLP (formyl methionine leucine phenylalanine peptide - or fMetLeuPhe - a specific chemoattractant of leucocytes). *K-252a actively and concentration-dependently (in a nanomolar range) antagonizes HMGB1-induced BASMC cell migration, while it does not interfere with cell migration induced by fMLP, whatever the concentration tested.*
Figure 2 - Activity of K-252a in a proliferation assay.
   K-252a inhibitory activity on HMGB1-induced bovine aorta smooth muscle cell (BASMC) proliferation. *K-252a antagonizes BASMC cell proliferation at all the concentrations tested in a time-dependent fashion and in a nM range.*
Figure 3 - circular dichroism of HMGB1 in the presence of K-252a.
Figure 4 - Inhibition by K-252a of mortality by LPS - induced endotoxemia. Treatment with K-252a shows a clear reversal of lethality induced by LPS in mice.

### Examples

### 1. Chemotaxis assay

Chemotaxis assays were performed using a well known and validated protocol (1). Modified Boyden chambers were used with filters having 5-8 µm pore size and treated with collagen I (100 µg/ml in 0.5 M acetic acid) and fibronectin (10 µg/ml, Roche). BASMC (bovine aorta smooth muscle cells) were cultured in serum-free DMEM and a sample of 20,000-40,000 cells was added to the upper well of a Boyden chamber.

K-252a was dissolved and diluted in the same serum-free medium and added to the lower well of the chamber. HMGB-1 (from calf thymus) concentration was 25 ng/ml, that one of fMLP was 0,1 µM while K-252a was 3, 10, 30, 100 nM. Overnight cell migration was allowed at 37+/-0.5 °C, then cells were scraped off and filters were fixed in methanol and stained in a solution of 10% crystal violet in 20% methanol. All experiments were performed at least twice in triplicate. Results are the mean +/- SD of the number of cells counted in 10 high power fields per filters and expressed as folds over control. Random cell migration, i.e. migration in the absence of chemoattractant, was given the arbitrary value of 100%. Statistical analysis was performed using Student's t test for pairwise comparisons of treatment, or an ANOVA model for the evaluation of treatments with increasing concentrations of a reagent. The results are shown in Fig. 1.

A chemotaxis assay as described above can be similarly performed using BAEC cells. Corresponding results are obtained.

### 2. Proliferation assay

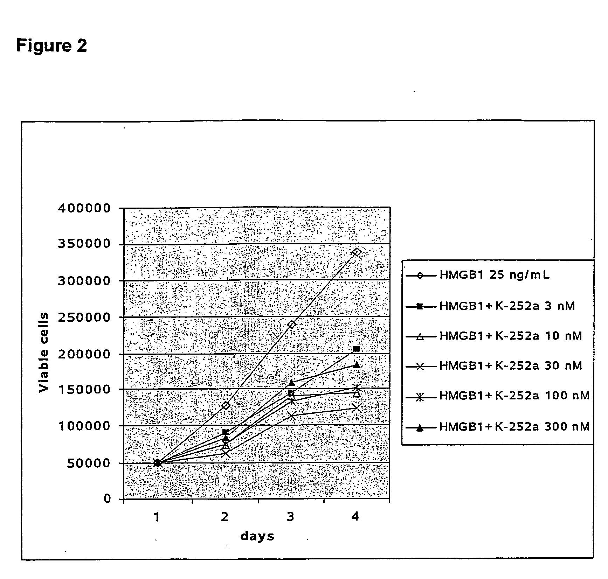

Proliferation assays were performed using an already described and validated method (1). BASMC cells (bovine aorta smooth muscle cells) were seeded in 6-well plates (10⁵ cells/well) and grown in RPMI medium supplemented with 20% FCS. After 24 h, the medium was replaced with serum-free RPMI and cell were then starved for 16 hours to synchronize the cell population. Vehicle (negative control or basal proliferation) or 25 ng/ml (1 nM) of HMGB1 (bacterially made) were added in the presence or in the absence of 3, 10, 30, 100 or 300 nM K-252a (dissolved and diluted in serum-free medium). Each experimental point represents the mean +/- SD of triplicate determinations. The experiment was repeated three times. BASMC cell proliferation was determined by detaching the cells from the plate at the indicated times (on days 1, 2, 3, and 4 of culturing) and counting the Trypan-blue excluding cells under the microscope. The results are shown in Fig. 2.

A proliferation assay as described above can be similarly performed using BAEC cells. Corresponding results are obtained.

### 3. Protein Binding experiments by Circular Dichroism (CD)

To check for protein binding of K-252a, a CD study was performed. All CD spectra were collected on a Jasco J710 spectropolarimeter equipped with a NesLab RTE111 thermal controller unity, using a quartz cylindrical cuvette with a 1 cm path length (Jasco). A scan speed of 20 nm/min, a bandwidth of 1 nm, and a resolution of 1 nm was always used.

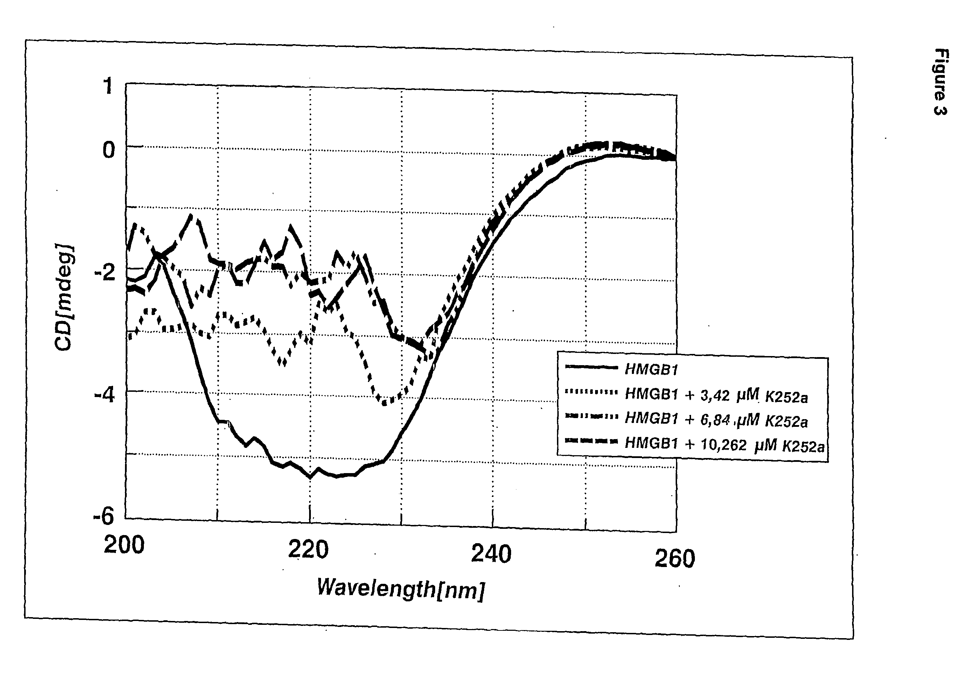

The addition of K-252a in concentrations of 3,42 µM, 6,84 µM and 10,26 µM to HMGB1 induces a great impact on the CD of HMGB1 in the range of 200 to about 235 nm (see Figure 3), but not in the range of about 235 to 260nm. The effect in the range of 200 to about 235 nm could, however, be attributed to the denaturating action of the solvent of K-252a (dimethylformamide, DMF) or to the interaction between K-252a and bacterial contaminants of HMGB1. The spectra are heavily disturbed more than likely due to DMF.

Assuming that a 1:1 stoichiometry binding takes place between HMGB1 and K-252a, the apparent K_{d} of the complex should be approximately 2 µM, a value which characterizes an extremely weak interaction. Therefore, direct interaction can not be considered the mechanism by which K-252a inhibits HMGB1-induced cell proliferation and migration. Fluorescence assays seem to confirm the absence of a direct interaction between K-252a and HMGB1.

K-252a is an inhibitor of tyrosine kinases (TrkA, TrkB, TrkC), of phosphokinases (PKA, PKC, PKG), and of further kinases (Raf kinase, Ras kinase, CaM kinase, MLC kinase, MAP kinase, MEK, ERK, JUN kinase, PI3Kγ). More than likely, K-252a does not inhibit HMGB1 at the receptor (RAGE) level either, but it may interfere with TrkA and/or with one of the kinases downstream of the HMGB1 interaction with RAGE or with Toll receptors.

### 4. Reversal by K-252a of LPS-induced endotoxemia in mice

Thirty-two male 6 to 7-week-old BALB/c mice were purchased from Charles River (Calco, Italy) and allowed to acclimatise for one week before use. On the day of the experiment, all mice were given an LD₇₀₋₉₀ (10.5 mg/kg i.p. in the right inguinal region) of lipopolysaccharide (LPS from Escherichia coli, strain 0111:B4 SIGMA, Lot 034154105), dissolved in 0.9% sterile saline. 15 min before LPS injection and 2, 12 and 24 h after LPS administration, 16 mice received K-252a (6,7 mg/kg i.p., 10 ml/kg, in the left inguinal region) dissolved in DMSO: sterile saline (8:92 v/v). The remaining 16 mice received the same volume of the vehicle alone (controls). Mice were observed for 7 consecutive days at least twice a day and deaths were recorded.

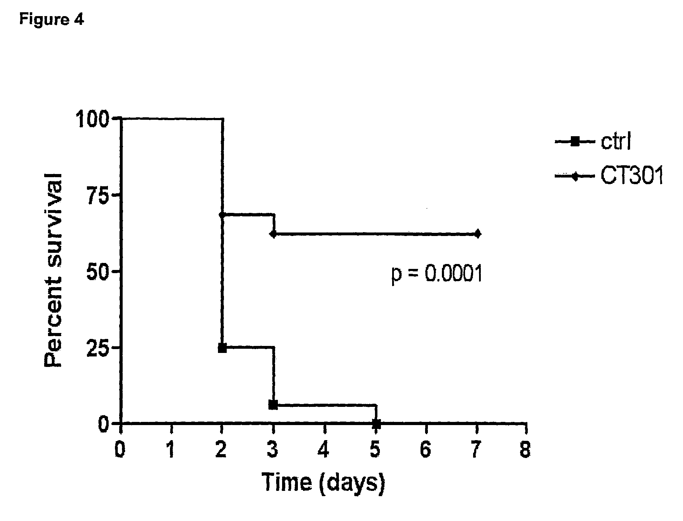

The results are shown in Figure 4. At the end of the observation period (7 days), 10 out of 16 mice (62,5%) treated with K-252a were still alive, while the last control mouse was found dead already in the course of the fifth day. Statistical analysis (Kaplan-Meier Survival Analysis) gives a "P" value of 0.0001.

### 5. Kinase inhibition profiling study

An analysis has been conducted with the aim of measuring the inhibitory activities of K-252a against 67 protein kinases at a concentration of 200 nM.
In particular, the target kinases were Tyrosine kinases and Serine/Threonine kinases.

### 5.2 Results

**Table 1 - Tyrosine kinases**

| Kinase | K-252a (200 nM) % Inhibition | Ref. % Inhibition | Ref. compound (nM) |
|---|---|---|---|
| ABL | -14.2 | 86.2 | Staurosporine (1000) |
| ARG | 0.1 | 89.6 | Staurosporine (3000) |
| TNK1 | 94.8 | 89.2 | Staurosporine (3) |
| ALK | 69.5 | 93.8 | Staurosporine (30) |
| AXL | 51.5 | 85.1 | Staurosporine (3000) |
| MER | 80.8 | 90.7 | Staurosporine (100) |
| CSK | 43.0 | 88.8 | Staurosporine (300) |
| EGFR | 16.4 | 91.4 | Staurosporine (10000) |
| HER2 | 7.7 | 65.6 | Staurosporine (10000) |
| HER4 | 39.4 | 94.1 | Staurosporine (10000) |
| EphA2 | 8.9 | 93.7 | Staurosporine (10000) |
| EphB2 | 0.4 | 83.7 | Staurosporine (1000) |
| EphB4 | 13.3 | 92.7 | Staurosporine (10000) |
| FAK | 53.5 | 90.4 | Staurosporine (100) |
| FGFR1 | 57.9 | 91.4 | Staurosporine (100) |
| FGFR2 | 67.2 | 96.7 | Staurosporine (100) |
| IGF1R | 40.6 | 98.6 | Staurosporine (3000) |
| INSR | 38.0 | 93.3 | Staurosporine (1000) |
| JAK1 | 83.1 | 82.8 | Staurosporine (30) |
| JAK2 | 99.5 | 96.3 | Staurosporine (3) |
| JAK3 | 100.4 | 97.8 | Staurosporine (3) |
| TYK2 | 98.1 | 94.5 | Staurosporine (3) |
| MET | 83.2 | 87.0 | Staurosporine (300) |
| RON | 5.2 | 81.6 | Staurosporine (10000) |
| FLT3 | 95.8 | 94.0 | Staurosporine (3) |
| CSFR | 55.7 | 89.1 | Staurosporine (30) |
| KIT | 87.3 | 96.7 | Staurosporine (10) |
| PDGFRα | 96.1 | 93.5 | Staurosporine (3) |
| PDGFRβ | 98.1 | 98.7 | Staurosporine (0.3) |
| RET | 94.2 | 88.1 | Staurosporine (30) |
| BLK | 83.5 | 94.9 | Staurosporine (30) |
| BRK | 35.8 | 87.4 | Staurosporine (10000) |
| FGR | 85.6 | 88.7 | Staurosporine (10) |
| FYN | 67.2 | 98.9 | Staurosporine (100) |
| HCK | 68.2 | 96.1 | Staurosporine (30) |
| LCK | 65.2 | 97.6 | Staurosporine (100) |
| LYNa | 58.9 | 95.2 | Staurosporine (100) |
| LYNb | 51.2 | 96.4 | Staurosporine (100) |
| SRC | 20.6 | 90.0 | Staurosporine (3000) |
| SRM | 0.4 | 74.0 | Staurosporine (10000) |
| YES | 65.1 | 94.1 | Staurosporine (30) |
| TrkA | 97.6 | 93.4 | Staurosporine (3) |
| TrkB | 98.2 | 67.3 | Staurosporine (0.3) |
| TrkC | 94.4 | 86.2 | Staurosporine (10) |
| FLT1 | 73.9 | 94.8 | Staurosporine (300) |
| KDR | 62.9 | 87.7 | Staurosporine (300) |

**Table 2 - Serine/Threonine kinases**

| Kinase | K-252a (200 nM) % Inhibition | Ref. % Inhibition | Ref. cpd (nM) |
|---|---|---|---|
| PKACα | 69.4 | 94.9 | Staurosporine (30) |
| PKCα | 64.4 | 97.8 | Staurosporine (30) |
| PKCε | 60.9 | 98.8 | Staurosporine (3) |
| PKCγ | 53.3 | 94.3 | Staurosporine (10) |
| CaMK4 | 0.0 | 88.4 | Staurosporine (3000) |
| CaMK2α | 83.9 | 92.9 | Staurosporine (30) |
| CHK1 | 92.8 | 91.1 | Staurosporine (10) |
| MAPKAPK2 | 44.3 | 90.5 | Staurosporine (1000) |
| CHK2 | 93.9 | 97.1 | Staurosporine (300) |
| CDK/CycA | 75.5 | 95.7 | Staurosporine (30) |
| Erk1 | 32.0 | 80.2 | 5-lodotubericidin (10000) |
| Erk2 | 14.2 | 62.1 | 5-lodotubericidin (10000) |
| JNK1 | 94.3 | 82.1 | JNK Inhibitor II (300) |
| JNK2 | 91.4 | 92.8 | JNK Inhibitor II (300) |
| p38α | -1.5 | 87.9 | SB202190 (300) |
| p38β | -0.2 | 55.7 | SB202190 (300) |
| AurA | 91.9 | 84.9 | Staurosporine (30) |
| IKKβ | 2.7 | 47.9 | Staurosporine (10000) |
| MAP2K3 | 100.0 | 84.5 | Staurosporine (30) |
| MAP2K7 | 68.2 | 85.8 | Staurosporine (3000) |
| IRAK4 | 51.9 | 95.9 | Staurosporine (10000) |

### 6. Conclusions

The very potent inhibitory effects shown by K-252a in *in vitro* models of cell migration and proliferation makes K-252a a promising drug candidate to be used in the systemic or local therapy of HMGB1-related diseases.

Based on these results, it is clear that HMGB1, both released by injured/dead endothelial cells or secreted by activated circulating macrophages and monocytes, is one of the most relevant targets of restenosis after surgical angioplasty and of several severe pathologies in the field of inflammation and immunity. The inhibition of its role and activities of a typical chemotactic chemokine mainly *in situ*, exactly where the mechanical traumatic injury is caused and the process leading to restenosis formation begins and develops, seems to indicate preventive/therapeutic activities of a specific HMGB1 antagonist.

For the same reason, the systemic administration of inhibitors of the pathological activities induced by HMGB1 looks like a promising therapeutic approach to cure a wide panel of systemic and local illnesses. It is demonstrated here that K-252a is a potent *in vitro* inhibitor of the two activities of HMGB1 mainly involved in restenosis induction and formation as well as in the triggering, sustaining and amplifying of local and systemic inflammatory and immune responses.

In fact, at concentrations which are in the nanomolar range, it inhibited HMGB1-induced cell migration (Fig. 1) and proliferation (Fig. 2). This, translated *in vivo*, means that K-252a as a therapeutic agent alone or released by a stent embedded/coated with K252 itself in the lesioned site should actively antagonize HMGB1 by blocking/inhibiting smooth muscle cell shift from the phenotype characterized by absence of cell division and migration to the synthetic phenotype which leads them to run and proliferate into the injured blood vessel endothelial wall, producing restenosis. Similarly, in the case of therapy of inflammatory and immunological pathologies triggered and sustained by HMGB1, the systemic administration of K-252a should inhibit/block the pathological cascade induced by this chemotactic chemokine, with beneficial effects on the onset and the course of the illness.

Moreover, the *in vivo* data obtained for the treatment with K-252a of mice affected by severe endotoxemia induced by LPS with K-252a, further support the results achieved with the above described *in vitro* data of cell migration and proliferation. In fact, the administration of 6,7 mg/kg i.p. of K-252a, for 4 times to mice bearing a severe LPS-induced endotoxemia, increase significantly (over 60%) the survival of the tested mice compared to the survival of the control mice. The control mice in fact do not survive over the fifth day. The *in vivo* results thus show a remarkable decrease in mortality in mice treated with K-252a, confirming that K-252a is a promising drug candidate to be used in the systemic or local treatment of HMGB1-related pathologies.

The mechanism by which K-252a inhibits the activity of HMGB1 seems to be kinase inhibition. Therefore, other kinase inhibitors, in particular known kinase inhibitors, e.g. of tyrosine kinase, such as TrkA, TrkB, TrkC, of phosphokinase, such as PKA, PKC, PKG, or/and of a further kinase, such as Raf kinase, Ras kinase, CaM kinase, MLC kinase, MAP kinase, MEK, ERK, JUN kinase, PI3Kγ, may also be suitable compounds for inhibiting the activity of HMGB1 *in vitro* and *in vivo.* Therefore, such kinase inhibitors may provide a promising therapeutic approach in diseases involving activity of released HMBG1.

### REFERENCES

1. Palumbo R., Sampaolesi M., De Marchis F., Tonlorenzi R., Colombetti S., Mondino A., Cossu G., Bianchi M. E. (2004) Extracellular HMGB1, a signal of tissue damage, induces mesoangioblast migration and proliferation. The Journal of Cell Biology, 164: 441-449.
2. Scaffidi P., Misteli T., Bianchi M.E. (2002) Release of chromatin protein HMGB1 by necrotic cells triggers inflammation, Nature. 418: 191-195.
3. Andersson, U., Erlandsson-Harris, H., Yang, H. and Tracey, K.J. (2002) HMGB1 as a DNA-binding cytokine. J. Leucocyte Biol., 72: 1084-1091
4. Agresti, A. and Bianchi, M.E. (2003) HMGB-proteins and gene expression Current Opin. In Genetics and Develop., 13: 170-178
5. Degryse, B., de Virgilio, M. (2003) The nuclear protein HMGB1, a new kind of chemokine ? FEBS Letters, 553: 11-17
6. Kase H., Iwahashi K., Matsuda Y. (1986) K-252a, a potent inhibitor of protein kinase C from microbial origin J. Antibiot. (Tokyo). 39: 1059-1065.
7. Nakanishi S., Yamada K., Kase H., Nakamura S., Nonomura Y. (1988) K-252a, a novel microbial product, inhibits smooth muscle myosin light chain kinase, J. Biol. Chem. 263: 6215-6219.
8. Delsite R. and Djakiew D. (1996) Anti-proliferative effect of the kinase inhibitor K-252a on human prostatic carcinoma cell lines, J. Androl. 17: 481-490.
**9.** Donovan M.J., Miranda R.C., Kraemer R., McCaffrey T.A., Tessarolo L., Mahadeo D., Sharif S., Kaplan D.R., Tsoulfas P., Parada L., et al. (1995) Neurotrophin and neurotrophin receptors in vascular smooth muscle cel/s. Regulation of expression in response to injury, Am. J. of Pathology. 147(2): 309-324.
**10.**Schmidt, A. M., Yan, S. F. and Stern, D. M. (2001) The multigland receptor RAGE as a progression factor amplifying immune and inflammatory responses J. Clin. Invest., 108: 949-955.
11.Bonaldi, T., Talamo, F., Scaffidi, P., Ferrera, D., Porto, A., Bachi, A., Rubartelli, A., Agresti, A. and Bianchi M.E. (2003) Monocytic cells hyperacetylate chromatin protein HMGB1 to redirect it towards Secretion The EMBO Journal, 22: 5551-5560
12.Friedman, S.G., Czura, C., J. and Tracey, K.J. (2003) The gesture life of high mobility group box 1 Current Opinion in Clinical Nutrition and Metabolica Care, 6: 283-287
13.Gardella, S., Andrei, C., Ferrera, D., Lotti, L.V., Torrisi, M.R., Bianchi, M.E. And Rubartelli, A. (2002) The nuclear protein HMGB1 is secreted by monocytes via a non-classical, vesicle- mediated secretory pathway. EMBO. Rep., 3: 995-1001

## Claims

1. Use of K-252a or/and a salt or a derivative thereof for the preparation of a medicament for the prevention or treatment of an HMGB1-associated pathology, which is triggered and/or sustained by HMGB1 and/or HMGB1 homologous proteins selected from restenosis, atherosclerosis or ischemia-reperfusion injury, wherein said derivative is a synthetic and/or chemically modified compound, which is a compound having substituents on the ring system selected from C₁-C₄ alkyl groups, a compound wherein the methyl ester group has been replaced by another ester group, an amide group or by H or a cation, and/or a compound wherein the N-atom in the cyclic amide group is substituted with a C₁-C₄ alkyl group and wherein the HMGB1 homologous proteins are HMGB2, HMGB3, HMG-1L10, HMG-4L or/and SP100-HMG.

2. Use of claim 1, wherein said HMGB1-associated pathology is associated with the non-acetylated form or/and the acetylated form of HMGB1 and associated with the non-acetylated form or/and the acetylated form of HMGB1 homologous proteins, wherein the HMGB1 homologous proteins are HMGB2, HMGB3, HMG-1L10, HMG-4L or/and SP100-HMG.

3. The use of claim 1 or 2, wherein the HMGB1-associated pathology is a pathological condition mediated by activation of the inflammatory cytokine cascade induced by HMGB1 or/and HMGB1 homologous proteins, wherein the HMGB1 homologous proteins are HMGB2, HMGB3, HMG-1L10, HMG-4L or/and SP100-HMG.

4. The use of claims 1-3 for blocking, retarding or impairing connective tissue regeneration in restenosis during or after angioplasty.

5. The use of any one of claims 1 to 4 in combination with a further agent.

6. The use of claim 5, wherein the further agent is capable of inhibiting early mediators of the inflammatory cytokine cascade.

7. The use of claim 6, wherein the further agent is an antagonist or inhibitor of a cytokine selected from the group consisting of TNF, IL-1α, IL-1β, IL-Rₐ, IL-8, MIP-1α, MIF1β, MIP-2. MIF and IL-6.

8. The use of claim 5, wherein the further agent is an antibody to RAGE, a nucleic acid or nucleic acid analogue capable of inhibiting RAGE expression, e.g. an antisense molecule, a ribozyme or a RNA interference molecule, or a small synthetic molecule antagonist of the HMGB1 interaction with RAGE or soluble RAGE (sRAGE), wherein said nucleic acid analogue is PNA or a DNA/PNA chimera or hybrid.

9. The use of claim 5, wherein the further agent is an antagonist or inhibitor of HMGB1.

10. The use of claim 9, wherein the antagonist or inhibitor is an antagonist or inhibitor of the non-acetylated or/and the acetylated form of HMGB1.

11. The use of claim 5, wherein the further agent is an inhibitor of the interaction of a Toll-like receptor (TLR), in particular of TLR2, TLR4, TLR7, TLR8 or/and TLR9, with HMGB1 or/and HMBG1 homologous proteins, preferably a monoclonal or polyclonal antibody, a nucleic acid or nucleic acid analogue capable of inhibiting TLR expression, e.g. an antisense molecule, a ribozyme or a RNA interference molecule, or a synthetic molecule having a size of less than 1000 Dalton, wherein said nucleic acid analogue is PNA or a DNA/PNA chimera or hybrid.

12. The use of claim 11, wherein the further agent is a known inhibitor of a Toll-like receptor, in particular of TLR2, TLR4, TLR7, TLR8 or/and TLR9, in particular a nucleic acid or nucleic acid analogue capable of inhibiting TLR expression, e.g. an antisense molecule, a ribozyme or a RNA interference molecule, wherein said nucleic acid analogue is PNA or a DNA/PNA chimera or hybrid.

13. The use of claim 5, wherein the further agent is the N-terminal lectine-like domain (D1) of thrombomodulin.

14. The use of claim 5, wherein the further agent is a synthetic double-stranded nucleic acid or nucleic acid analogue molecule with a bent shape structure, wherein said nucleic acid analogue molecule is PNA or a DNA/PNA chimera or hybrid.

15. The use of claim 14, wherein the synthetic double-stranded nucleic acid or nucleic acid analogue molecule is a double-stranded bent or cruciform DNA, PNA or DNA/PNA chimera or hybrid.

16. The use according to any one of claims 1 to 15, wherein K-252a or/and a salt or a derivative thereof is reversibly immobilised on the surface of a medical device.

17. The use of claim 16, wherein K-252a or/and a salt or a derivative thereof is coated on or embedded in the surface of the medical device.

18. The use of claims 16 or 17, wherein the medical device is selected from surgical instruments, implants, catheters or stents.

19. A medical device reversibly coated or/and embedded with K-252a or/and a salt or a derivative thereof, wherein said derivative is a synthetic and/or chemically modified compound, which is a compound having substituents on the ring system selected from C₁-C₄ alkyl groups, a compound wherein the methyl ester group has been replaced by another ester group, an amide group or by H or a cation, and/or a compound wherein the N-atom in the cyclic amide group is substituted with a C₁-C₄ alkyl group.

20. The medical device of claim 19, wherein the medical device is selected from surgical instruments, implants, catheters or stents.

## Patentansprüche

1. Die Verwendung von K-252a oder/und eines Salzes oder eines Derivats davon zur Herstellung eines Medikaments zur Prävention oder Behandlung einer mit HMGB1 verbundenen Symptomatik, die durch HMGB1 und/oder durch HMGB1 homologe Proteine ausgelöst und/oder erhalten wird, die aus Restenose, Atherosklerose oder einer Ischämie-Reperfusionserkrankung ausgewählt wird, wobei das Derivat eine synthetische und/oder chemisch modifizierte Verbindung ist, welche Substituenten am Ringsystem enthält, die aus C₁-C₄-Alkylgruppen ausgewählt werden, in der die Methylestergruppe durch eine andere Estergruppe, eine Amidgruppe oder durch Wasserstoff oder ein Kation ersetzt wurde und/oder in der das N-Atom in der zyklischen Amidgruppe durch eine C₁-C₄-Alkylgruppe ersetzt wurde, und in der die HMGB1 homologen Proteine HMGB2, HMGB3, HMG-1L10, HMG-4L oder/und SP100-HMG sind.

2. Die Verwendung nach Anspruch 1, in der die mit HMGB1 verbundene Symptomatik mit der nicht-azetylierten Form oder/und der azetylierten Form von HMGB1 und mit der nicht-azetylierten Form oder/und der azetylierten Form der HMGB1 homologen Proteine verbunden ist, in der die HMGB1 homologen Proteine HMGB2, HMGB3, HMG-1L10, HMG-4L oder/und SP100-HMG sind.

3. Die Verwendung nach Anspruch 1 oder 2, in der die mit HMGB1 verbundene Symptomatik ein pathologischer Zustand ist, der durch die Aktivierung der entzündlichen Zytokinkaskade vermittelt wird, die durch HMGB1 oder/und HMGB1 homologe Proteine induziert wird, in der die HMGB1 homologen Proteine HMGB2, HMGB3, HMG-1L10, HMG-4L oder/und SP100-HMG sind.

4. Die Verwendung nach einem der Ansprüche 1 bis 3 zur Blockierung, Retardierung oder Verminderung der Regenerierung des Bindegewebes bei der Restenose während oder nach einer Angioplastie.

5. Die Verwendung nach einem der Ansprüche 1 bis 4 in Kombination mit einem weiteren Wirkstoff.

6. Die Verwendung nach Anspruch 5, in der der weitere Wirkstoff zur Inhibierung früher Mediatoren der entzündlichen Zytokinkaskade in der Lage ist.

7. Die Verwendung nach Anspruch 6, in der der weitere Wirkstoff ein Antagonist oder Inhibitor eines Zytokins ist, das aus der Gruppe bestehend aus TNF, IL-1α, IL1β, IL-Rₐ, IL-8, MIP-1α, MIF-1β, MIP-2, MIF und IL-6 ausgewählt wird.

8. Die Verwendung nach Anspruch 5, in der der weitere Wirkstoff ein Antikörper gegen RAGE, eine Nukleinsäure oder ein Nukleinsäureanalog ist, das die RAGE-Expression inhibieren kann, wie z. B. ein Antisense-Molekül, ein Ribozym oder ein RNA-Interferenz-Molekül oder ein kleiner synthetischer molekularer Antagonist der HMGB1 Interaktion mit RAGE oder löslichem RAGE (sRAGE), wobei das Nukleinsäureanalog PNA oder eine DNA/PNA-Chimäre oder ein DNA/PNA-Hybrid ist.

9. Die Verwendung nach Anspruch 5, in der der weitere Wirkstoff ein Antagonist oder Inhibitor von HMGB1 ist.

10. Die Verwendung nach Anspruch 9, in der der Antagonist oder Inhibitor ein Antagonist oder Inhibitor der nicht-azetylierten oder/und azetylierten Form von HMGB1 ist.

11. Die Verwendung nach Anspruch 5, in der der weitere Wirkstoff ein Inhibitor der Interaktion eines Toll-like Rezeptors (TLR), insbesondere von TLR2, TLR4, TLR7, TLR8 und/oder TLR9 mit HMGB1 oder/und HMGB1 homologer Proteine ist, bevorzugt ein monoklonaler oder polyklonaler Antikörper, eine Nukleinsäure oder ein Nukleinsäureanalog, das die TLR Expression inhibieren kann wie z. B. ein Antisense-Molekül, ein Ribozym oder ein RNA-Interferenz-Molekül oder ein synthetisches Molekül einer Größe von weniger als 1000 Dalton, wobei das Nukleinsäureanalog PNA oder eine DNA/PNA-Chimäre oder ein DNA/PNA-Hybrid ist.

12. Die Verwendung nach Anspruch 11, in der der weitere Wirkstoff ein bekannter Inhibitor eines Toll-like Rezeptors ist, insbesondere von TRL2, TLR4, TLR7, TLR8 und/oder TLR9, insbesondere eine Nukleinsäure oder ein Nukleinsäureanalog, das die TLR Expression inhibieren kann wie z. B. ein Antisense-Molekül, ein Ribozym oder ein RNA-Interferenz-Molekül, wobei das Nukleinsäureanalog PNA oder eine DNA/PNA-Chimäre oder ein DNA/PNA-Hybrid ist.

13. Die Verwendung nach Anspruch 5, in der der weitere Wirkstoff die N-terminale Lektin-ähnliche Domäne (D1) von Thrombomodulin ist.

14. Die Verwendung nach Anspruch 5, in der der weitere Wirkstoff eine synthetische doppelsträngige Nukleinsäure oder ein nukleinsäureanaloges Molekül mit einer gekrümmt förmigen Struktur ist, wobei das nukleinsäureanaloge Molekül PNA oder eine DNA/PNA-Chimäre oder ein DNA/PNA-Hybrid ist.

15. Die Verwendung nach Anspruch 14, in der das synthetische doppelsträngige Nukleinsäure oder das nukleinsäureanaloge Molekül eine doppelsträngige gekrümmte oder kruziforme DNA, PNA oder DNA/PNA-Chimäre oder ein DNA/PNA-Hybrid ist.

16. Die Verwendung nach einem der Ansprüche 1 bis 15, in der K-252a oder/und ein Salz oder Derivat davon auf der Oberfläche eines medizintechnischen Geräts reversibel immobilisiert ist.

17. Die Verwendung nach Anspruch 16, in der K-252a oder/und ein Salz oder Derivat davon auf der Oberfläche eines medizintechnischen Geräts aufgebracht oder eingebettet ist.

18. Die Verwendung nach Anspruch 16 oder 17, in der das medizintechnische Gerät aus chirurgischen Instrumenten, Implantaten, Kathetern oder Stents ausgewählt wird.

19. Medizintechnisches Gerät, das reversibel mit K-252a oder/und einem Salz oder Derivat davon beschichtet oder in dieses eingebettet ist, wobei das Derivat eine synthetische und/oder chemisch modifizierte Verbindung ist, welche Substituenten am Ringsystem enthält, die aus C₁-C₄-Alkylgruppen ausgewählt werden, in der die Methylestergruppe durch eine andere Estergruppe, eine Amidgruppe oder durch Wasserstoff oder ein Kation ersetzt wurde und/oder in der das N-Atom in der zyklischen Amidgruppe durch eine C₁-C₄-Alkylgruppe ersetzt wurde.

20. Das medizintechnische Gerät nach Anspruch 19, wobei das medizintechnische Gerät aus chirurgischen Instrumenten, Implantaten, Kathetern oder Stents ausgewählt wird.

## Revendications

1. Utilisation de K-252a ou/et d'un sel ou d'un dérivé de celui-ci, dans la préparation d'un médicament pour la prévention ou le traitement d'une pathologie associée à HMGB 1, qui est provoquée et/ou soutenue par les protéines homologues HMGB 1 et/ou HMGB1, choisie parmi la resténose, l'athérosclérose ou la lésion ischémique-de reperfusion, dans laquelle ledit dérivé est un composé synthétique et/ou modifié chimiquement, qui est un composé ayant des substituants sur le système de cycle choisis parmi des groupes alkyle en C₁-C₄, un composé dans lequel le groupe méthyl-ester a été remplacé par un autre groupe ester, un groupe amide ou par un H ou un cation, et/ou un composé dans lequel l'atome N dans le groupe amide cyclique est substitué par un groupe alkyle en C₁-C₄, et dans lequel les protéines homologues HMBG1 sont HMGB2, HMGB3, HMG-IL10, HMG-4L ou/et SP100-HMG.

2. Utilisation selon la revendication 1, dans laquelle ladite pathologie associée à HMGB 1 est associée à la forme non acétylée ou/et à la forme acétylée de HMGB 1 et associée à la forme non acétylée ou/et à la forme acétylée des protéines homologues HMGB1, dans laquelle les protéines homologues HMBG1 sont HMGB2, HMGB3, HMG-1L10, HMG-4L ou/et SP100-HMG.

3. Utilisation selon la revendication 1 ou la revendication 2, dans laquelle la pathologie associée à HMGB1 est un état pathologique provoqué par l'activation de la cascade de cytokine inflammatoire induite par HMGB1 ou/et les protéines homologues HMGB1, dans laquelle les protéines homologues HMBG1 sont HMGB2, HMGB3, HMG-1L10, HMG-4L ou/et SP100-HMG.

4. Utilisation selon l'une quelconque des revendications 1-3, pour bloquer, retarder ou altérer la régénération des tissus connectifs en resténose pendant ou après l'angioplastie.

5. Utilisation selon l'une quelconque des revendications 1 à 4, en combinaison avec un autre agent.

6. Utilisation selon la revendication 5, dans laquelle l'autre agent est capable d'inhiber les médiateurs précoces de la cascade de cytokine inflammatoire.

7. Utilisation selon la revendication 6, dans laquelle l'autre agent est un antagoniste ou un inhibiteur d'une cytokine choisie dans le groupe constitué de TNF, IL-1α, IL-1β, IL-R₈, IL-8, MIP-1α, MIF1β, MIP-2, MIF et IL-6.

8. Utilisation selon la revendication 5, dans laquelle l'autre agent est un anticorps contre le RAGE, un acide nucléique ou un analogue d'acide nucléique capable d'inhiber l'expression du RAGE, par exemple une molécule anti-sens, un ribozyme ou une molécule d'interférence d'ARN, ou un antagoniste de petite molécule synthétique de l'interaction de HMGB 1 avec RAGE ou un RAGE soluble (sRAGE), dans laquelle ledit analogue d'acide nucléique est un ANP ou un chimère ou hybride d'ADN/ANP.

9. Utilisation selon la revendication 5, dans laquelle l'autre agent est un antagoniste ou un inhibiteur de HMGB 1.

10. Utilisation selon la revendication 9, dans laquelle l'antagoniste ou l'inhibiteur est un antagoniste ou un inhibiteur de la forme non acétylée ou/et acétylée de HMGB 1.

11. Utilisation selon la revendication 5, dans laquelle l'autre agent est un inhibiteur de l'interaction d'un récepteur de type Toll (TLR), en particulier de TLR2, TLR4, TLR7, TLR8 ou/et TLR9, avec HMGB1 ou/et des protéines homologues HMBG1, de préférence un anticorps monoclonal ou polyclonal, un acide nucléique ou un analogue d'acide nucléique capable d'inhiber l'expression de TLR, par exemple une molécule anti-sens, un ribozyme ou une molécule d'interférence d'ARN, ou une molécule synthétique ayant une taille inférieure à 1000 Daltons, dans laquelle ledit analogue d'acide nucléique est un ANP ou une chimère ou hybride d'ADN/ANP.

12. Utilisation selon la revendication 11, dans laquelle l'autre agent est un inhibiteur connu d'un récepteur de type Toll, en particulier de TLR2, TLR4, TLR7, TLR8 ou/et TLR9, en particulier un acide nucléique ou un analogue d'acide nucléique capable d'inhiber l'expression de TLR, par exemple une molécule anti-sens, un ribozyme ou une molécule d'interférence d'ARN, dans laquelle ledit analogue d'acide nucléique est un ANP ou une chimère ou hybride d'ADN/ANP.

13. Utilisation selon la revendication 5, dans laquelle l'autre agent est le domaine semblable à la lectine à terminal N (D1) de la thrombomoduline.

14. Utilisation selon la revendication 5, dans laquelle l'autre agent est un acide nucléique double-brin synthétique ou une molécule d'analogue d'acide nucléique ayant une structure en forme pliée, dans laquelle ladite molécule d'analogue d'acide nucléique est un ANP ou une chimère ou hybride d'ADN/ANP.

15. Utilisation selon la revendication 14, dans laquelle l'acide nucléique double-brin synthétique ou la molécule d'analogue d'acide nucléique est un ADN double-brin plié ou cruciforme, un ANP ou une chimère ou hybride d'ADN/ANP.

16. Utilisation selon l'une quelconque des revendications 1 à 15, dans laquelle le K-252a ou/et un sel ou un dérivé de celui-ci est immobilisé de façon réversible sur la surface d'un dispositif médical.

17. Utilisation selon la revendication 16, dans laquelle le K-252a ou/et un sel ou un dérivé de celui-ci est revêtu sur ou intégré dans la surface du dispositif médical ou intégré dans celui-ci.

18. Utilisation selon les revendications 16 ou 17, dans laquelle le dispositif médical est choisi parmi les instruments chirurgicaux, les implants, les cathéters ou les stents.

19. Dispositif médical revêtu ou/et intégré de façon réversible avec le K-252a ou/et un sel ou un dérivé de celui-ci, dans lequel ledit dérivé est un composé synthétique et/ou modifié chimiquement, qui est un composé ayant des substituants sur le système de cycle choisis parmi les groupes alkyle en C₁-C₄, un composé dans lequel le groupe méthyl-ester a été remplacé par un autre groupe ester, un groupe amide ou par un H ou un cation, et/ou un composé dans lequel l'atome N dans le groupe amide cyclique est substitué par un groupe alkyle en C₁-C₄.

20. Dispositif médical selon la revendication 19, dans lequel le dispositif médical est choisi parmi les instruments chirurgicaux, les implants, les cathéters ou les stents.
